Europäisches Patentamt
European Patent Office
Office européen des brevets

(19) (11) Publication number: 0 277 751 A2

(12) **EUROPEAN PATENT APPLICATION**

(21) Application number: 88300641.3

(22) Date of filing: 26.01.88

(51) Int. Cl.4: C12N 15/00 , C12N 9/64 , A61K 37/54

Claims for the following Contracting State: ES.

The title of the invention has been amended (Guidelines for Examination in the EPO, A-III, 7.3).

(30) Priority: 28.01.87 GB 8701811

(43) Date of publication of application: 10.08.88 Bulletin 88/32

(84) Designated Contracting States: AT BE CH DE ES FR GB GR IT LI LU NL SE

(71) Applicant: BEECHAM GROUP PLC
Beecham House Great West Road
Brentford Middlesex TW8 9BD(GB)

(72) Inventor: Browne, Michael Joseph Beecham Pharmaceuticals
Great Burgh Yew Tree Bottom Road
Epsom Surrey KT18 5XQ(GB)
Inventor: Robinson, Jeffery Beecham Pharmaceuticals
Great Burgh Yew Tree Bottom Road
Epsom Surrey KT18 5XQ(GB)

(74) Representative: Valentine, Jill Barbara et al
Beecham Pharmaceuticals Patents & Trade Marks Dept. Great Burgh Yew Tree Bottom Road
Epsom Surrey KT18 5XQ(GB)

(54) **Modified fibrinolytic enzyme.**

(57) A fibrinolytically active tissue-type plasminogen activator which has been modified:
(a) in the region of the growth factor domain, and
(b) to provide an amino acid other than arginine or lysine at position 275.

EP 0 277 751 A2

## Novel Compounds

The present invention relates to a modified fibrinolytic enzyme in particular modified tissue-type plasminogen activator, its preparation, pharmaceutical compositions containing it and its use in the treatment of thrombotic disease.

The sequence of amino acids making up the enzyme tissue-type plasminogen activator (t-PA) and the nucleotide sequence for the cDNA which codes for t-PA are known (see Pennica et al, 1983; Nature, 301, 214). Tissue-type plasminogen activator is known to have fibrinolytic activity.

It has been shown (Bànyai, L. et al, 1983; FEBS Lett., 163, 37)that a part of the t-PA enzyme shows structural homology with human and murine epidermal growth factors. This region from amino acid residues 44 to 91 has been termed the "growth factor domain". The genetic information which codes for the major part of this domain, residues 51 to 86 inclusive, and partially codes for residues 50 and 87, lies on a single exon (Ny, T. et al, 1984; Proc. Nat. Acad. Sci. U.S.A., 81, 5355).

EP-A-0207589 discloses t-PA modified in the region of the growth factor domain, in particular by the deletion of amino acid residues 51 to 87 inclusive of native t-PA. EP-A-0233013 discloses t-PA comprising an amino acid other than arginine or lysine at position 275, in particular glutamine.

The applicants have now identified modified forms of the t-PA enzyme which retain fibrinolytic activity.

According to the present invention there is provided a fibrinolytically active tissue-type plasminogen activator which has been modified:

(a) in the region of the growth factor domain, and

(b) to provide an amino acid other than arginine or lysine at position 275.

Suitable growth factor domain modifications (modification (a)) may include removal or deletion of certain amino acid residues, or replacement of one or more amino acid residues with different amino acid residues.

In a preferred aspect, the modification (a) comprises the deletion of all or part of the growth factor domain.

In another preferred aspect, the modification (a) occurs in the region from amino acid residues 51 to 87 inclusive, in particular by deletion of that region.

Preferred amino acids for substitution at position 275 (modification (b)) are those similar to arginine in hydrophilicity and size. Thus histidine, threonine, serine, asparagine, aspartic acid, glutamine and glutamic acid are preferred. Other suitable amino acids include alanine and glycine.

In a further preferred aspect, modification (b) is such that the amino acid at position 275 is glutamine.

As used herein, the term tissue-type plasminogen activator denotes a plasminogen activator of the group having the immunological properties defined for t-PA at the XXVIII Meeting of the International Committee on Thrombosis and Haemostasis, Bergamo, Italy, 27 July 1982.

The amino acid sequence of various forms of t-PA are known. The abovementioned Nature 1983 reference discloses the sequence for the L-chain and the mature S-chain forms of t-PA, also discussed by Vehar et.al., Biotechnology, 1984, 2, 1051-7 in which the processing of initially formed t-PA by removal of a pro-sequence to give the S-chain form is reported. Pohl et.al., FEBS letters, 1984, Vol. 168 No.1, 29-32, refers to the N-terminal multiplicity of t-PA and discloses the U-chain form. The numbering system for the amino acid sequence of t-PA used herein is that described in the Nature 1983 reference for mature (S-chain) t-PA in which the N-terminal serine is numbered 1. By this system, L-chain t-PA has an N-terminal glycine residue at position -3 and U-chain t-PA has an N-terminal valine at position 4. It is understood that the tissue-type plasminogen activator modified in accordance with the present invention encompasses all such variant forms.

The modified t-PA of the invention may be derivatised to provide pharmaceutically useful conjugates analogous to known t-PA-containing conjugates, for example:

(a) an enzyme-protein conjugate as disclosed in EP-A-O 155 388, in which the catalytic site on the enzyme which is responsible for fibrinolytic activity is blocked by a human protein attached thereto by way of a reversible linking group;

(b) an enzyme-protein conjugate as disclosed in EP-A-O 152,736, comprising at least one optionally blocked fibrinolytic enzyme linked by way of a site other than the catalytic site responsible for fibrinolytic activity to at least one human protein;

(c) a protein-polymer conjugate as disclosed in EP-A-0183503 comprising a pharmaceutically useful protein linked to at least one water soluble polymer by means of a reversible linking group; or

(d) an enzyme conjugate as disclosed in EP-A-0184363 comprising a plurality of fibrinolytic enzymes linked together through the active centres thereof by means of a removable blocking group.

The modified t-PA of the invention may take the place of t-PA as the enzyme or (human) protein

component, as appropriate, of any of the conjugates described above.

The modified t-PA of the invention or conjugate thereof can be further derivatised such that any catalytic site essential for fibrinolytic activity is optionally blocked by a removable blocking group.

The above mentioned derivatives of the modified t-PA may be used in any of the methods and compositions described hereinafter for the modified t-PA itself.

As used herein the expression 'removable blocking group' includes groups which are removable by hydrolysis at a rate such that the pseudo-first order rate constant for hydrolysis is in the range of $10^{6}$ sec$^{1}$ to $10^{2}$ sec$^{1}$ in isotonic aqueous media at pH 7.4 at 37°C.

Such blocking groups are described in European Patent No.0009879 and include acyl groups such as optionally substituted benzoyl or optionally substituted acryloyl.

Suitable optional substituents for benzoyl blocking groups include halogen, $C_{1-6}$ alkyl, $C_{1-6}$ alkoxy, $C_{1-6}$alkanoyloxy, $C_{1-6}$ alkanoylamino, amino or p-guanidino.

Suitable optional substituents for acryloyl blocking groups include $C_{1-6}$ alkyl, furyl, phenyl or $C_{1-6}$alkylphenyl.

In a further aspect, the invention provides a process for preparing modified tissue-type plasminogen activator according to the invention which process comprises expressing DNA encoding said modified tissue-type plasminogen activator in a recombinant host cell and recovering the modified tissue-type plasminogen activator product.

The modification of the t-PA can therefore be carried out by conventional genetic engineering techniques in which the cDNA which codes for t-PA is modified and the modified cDNA expressed in a prokaryote or eukaryote host.

The DNA polymer comprising a nucleotide sequence that encodes the modified t-PA also forms part of the invention.

The process of the invention may be performed by conventional recombinant techniques such as described in Maniatis et. al., Molecular Cloning - A Laboratory Manual; Cold Spring Harbor, 1982.

In particular, the process may comprise the steps of:

i) preparing a replicable expression vector capable, in a host cell, of expressing a DNA polymer comprising a nucleotide sequence that encodes said modified tissue-type plasminogen activator;

ii) transforming a host cell with said vector;

iii) culturing said transformed host cell under conditions permitting expression of said DNA polymer to produce said modified tissue-type plasminogen activator; and

iv) recovering said modified tissue-type plasminogen activator.

The invention also provides a process for preparing the DNA polymer by the condensation of appropriate mono-, di-or oligomeric nucleotide units.

The preparation may be carried out chemically enzymatically, or by a combination of the two methods, in vitro or in vivo as appropriate. Thus, the DNA polymer may be prepared by the enzymatic ligation of appropriate DNA fragments, by conventional methods such as those described by D. M. Roberts et al in Biochemistry 1985, 24, 5090-5098. The DNA fragments may be obtained by the digestion of DNA containing the required sequences of nucleotides with appropriate restriction enzymes, by chemical synthesis, by enzymatic polymerisation, or by a combination of these methods.

Digestion with restriction enzymes may be performed in an appropriate buffer at a temperature of 20°-70°C, generally in a volume of 50µl or less with 0.1-10µg DNA.

Enzymatic polymerisation of DNA may be carried out in vitro using a DNA polymerase such as DNA polymerase I (Klenow fragment) in an appropriate buffer containing the nucleoside triphosphates dATP, dCTP, dGTP and dTTP as required at a temperature of 10°-37°C, generally in a volume of 5µl or less. Enzymatic ligation of DNA fragments may be carried out using a DNA ligase such as T4 DNA ligase in an appropriate buffer, such as 0.05M Tris (pH 7.4), 0.01M $MgCl_2$, 0.01m dithiothreitol, 1mM spermidine, 1mM ATP and 0.1mg/ml bovine serum albumin, at a temperature of 4°C to ambient, generally in a volume of 50µl or less. The chemical synthesis of the DNA polymer or fragments may be carried out by conventional phosphotriester, phosphite or phosphoramidite chemistry, using solid phase techniques such as those described in 'Chemical and Enzymatic Synthesis of Gene Fragments - A Laboratory Manual' (ed. H.G. Gassen and A. Lang), Verlag Chemie, Weinheim (1982),or in other scientific publications, for example M.J. Gait, H.W.D. Matthes, M. Singh, B.S. Sproat, and R.C. Titmas, Nucleic Acids Research, 1982, 10, 6243; B.S. Sproat and W. Bannwarth, Tetrahedron Letters, 1983, 24, 5771; M.D. Matteucci and M.H Caruthers, Tetrahedron Letters, 1980, 21, 719; M.D. Matteucci and M.H. Caruthers, Journal of the American Chemical Society, 1981, 103, 3185; S.P. Adams et al., Journal of the American Chemical Society,1983, 105, 661; N.D. Sinha, J. Biernat, J. McMannus, and H. Koester, Nucleic Acids Research, 1984, 12, 4539; and H.W.D. Matthes et al., EMBO Journal, 1984, 3, 801.

DNA polymer which encodes the modified t-PA may be prepared by site-directed mutagenesis of the cDNA which codes for tissue-type plasminogen activator, by conventional methods such as those described by G. Winter et al in Nature 1982, 299, 756-758 or by Zoller and Smith 1982; Nucl. Acids Res., 10, 6487-6500, or deletion mutagenesis such as described by Chan and Smith in Nucl. Acids Res., 1984, 12, 2407-2419 or by G. Winter et al in Biochem. Soc. Trans., 1984, 12, 224-225.

In particular, the preparation may be carried out by the simultaneous or sequential application of the mutagenesis method disclosed in EP-A-0207589 and the site-directed mutagenesis of cDNA coding for t-PA at the codon formed by the nucleotides 1012-1014 coding for arginine-275 as described in EP-A-0233013. Thus, the preparation may comprise the simultaneous mutagenesis of the cDNA coding for native t-PA in the region coding for the growth factor domain (to provide modification (a)) and at the codon encoding arginine-275 (to provide modification (b)). Alternatively, the preparation may comprise the mutagenesis of DNA which codes for t-PA having one of the modifications (a) and (b), to provide the other modification.

Alternatively and preferably, the DNA polymer may be prepared by the enzymatic ligation of a first DNA fragment which encodes a portion of t-PA encompassing modification (a) with a second DNA fragment which encodes a portion of t-PA encompassing modification (b). The DNA fragments may be obtained by the digestion of DNA polymers coding for t-PA containing modification (a) and modification (b) respectively. Conveniently the same restriction site is selected, lying between the sites or potential sites of both modifications, for both DNA polymers, so that the resulting fragments may be ligated to restore the t-PA DNA sequence but including modifications (a) and (b). A convenient restriction site is the NarI site.

The expression of the DNA polymer encoding the modified t-PA in a recombinant host cell may be carried out by means of a replicable expression vector capable, in the host cell, of expressing the DNA polymer. The expression vector is novel and also forms part of the invention.

The replicable expression vector may be prepared in accordance with the invention, by cleaving a vector compatible with the host cell to provide a linear DNA segment having an intact replicon, and combining said linear segment with one or more DNA molecules which, together with said linear segment encode the modified tissue-type plasminogen activator, such as the DNA polymer encoding the modified t-PA or fragments thereof, under ligating conditions.

Thus, the DNA polymer may be preformed or formed during the construction of the vector, as desired.

The choice of vector will be determined in part by the host cell, which may be prokaryotic or eukaryotic. Suitable vectors include plasmids, bacteriophages, cosmids and recombinant viruses.

The preparation of the replicable expression vector may be carried out conventionally with appropriate enzymes for restriction, polymerisation and ligation of the DNA, by procedures described in, for example, Maniatis et al cited above. Restriction, polymerisation and ligation may be performed as described above for the preparation of the DNA polymer.

The recombinant host cell is prepared, in accordance with the invention, by transforming a host cell with a replicable expression vector of the invention under transforming conditions. Suitable transforming conditions are conventional and are described in, for example, Maniatis et al cited above, or "DNA Cloning" Vol. II, D.M. Glover ed., IRL Press Ltd, 1985.

The choice of transforming conditions is determined by the host cell. Thus, a bacterial host such as E. coli may be treated with a solution of $CaCl_2$ (Cohen et al, Proc. Nat. Acad. Sci., 1973, 69, 2110) or with a solution comprising a mixture of RbCl, $MnCl_2$, potassium acetate and glycerol, and then with 3-[N-morpholino]propane-sulphonic acid, RbCl and glycerol. Mammalian cells in culture may be transformed by calcium co-precipitation of the vector DNA onto the cells. The invention also extends to a host cell transformed with a replicable expression vector of the invention.

Culturing the transformed host cell under conditions permitting expression of the DNA polymer is carried out conventionally, as described in, for example, Maniatis et al and "DNA Cloning" cited above. Thus, preferably the cell is supplied with nutrient and cultured at a temperature below 45°C

The modified t-PA expression product is recovered by conventional methods according to the host cell. Thus, where the host cell is bacterial, such as E. coli it may be lysed physically, chemically or enzymatically and the protein product isolated from the resulting lysate. Where the host cell is mammalian, the product may generally be isolated from the nutrient medium. The DNA polymer may be assembled into vectors designed for isolation of stable transformed mammalian cell lines expressing the modified t-PA; e.g. bovine papillomavirus vectors (DNA cloning Vol.II D.M. Glover Ed. IRL Press 1985; Kaufman, R.J. et al, Molecular and Cellular Biology 5, 1750-1759, 1985; Pavlakis G.N. and Hamer, D.H., Proceedings of the National Academy of Sciences (USA) 80, 397-401, 1983; Goeddel, D.V. et al., European Patent Application No. 0093619, 1983).

It will be appreciated that, depending upon the host cell, the modified t-PA prepared in accordance with the invention may be glycosylated to varying degrees. Furthermore, as observed by Pohl et.al., Biochemistry, 1984, 23, 3701-3707, varying degress of glycosylation may also be found in unmodified, naturally occurring t-PA. The modified t-PA of the invention is understood to include such glycosylated variations.

The modified t-PA of the invention is suitably administered in the form of a pharmaceutical composition.

Accordingly the present invention also provides a pharmaceutical composition comprising modified t-PA of the invention in combination with a pharmaceutically acceptable carrier.

The compositions according to the invention may be formulated in accordance with routine procedures as pharmaceutical compositions adapted for intravenous administration to human beings.

Typically compositions for intravenous administration are solutions of the sterile enzyme in sterile isotonic aqueous buffer. Where necessary the composition may also include a solubilising agent to keep the modified t-PA in solution and a local anaesthetic such as lignocaine to ease pain at the site of injection. Generally, the modified t-PA will be supplied in unit dosage form for example as a dry powder or water free concentrate in a hermetically sealed container such as an ampoule or sachette indicating the quantity of protein in activity units. Where the modified t-PA includes a removable blocking group an indication of the time within which the free protein will be liberated may be driven. Where the protein is to be administered by infusion it will be dispensed with an infusion bottle containing sterile pharmaceutical grade 'Water for Injection' or saline. Where the protein is to be administered by injection, it is dispensed with an ampoule of sterile water for injection or saline. The injectable or infusable composition will be made up by mixing the ingredients prior to administration. The quantity of material administered will depend upon the amount of fibrinolysis required and the speed with which it is required, the seriousness of the thromboembolic condition and position and size of the clot. The precise dose to be employed and mode of administration must per force in view of the nature of the complaint be decided according to the circumstances by the physician supervising treatment. However, in general, a patient being treated for a mature, fresh or nascent thrombus will generally receive a daily dose of from 0.01 to 10 mg/kg of body weight either by injection in for example up to five doses or by infusion.

Within the above indicated dosage range, no adverse toxicological effects are indicated with the compounds of the invention.

Accordingly, in a further aspect of the invention there is provided a method of treating thrombotic diseases, which comprises administering to the sufferer an effective non-toxic amount of modified t-PA of the invention.

The invention also provides a modified t-PA of the invention for use as an active therapeutic substance and, in particular, for use in the treatment of thrombotic diseases.

The following Examples illustrate the invention.

## Example 1

5'd(TCCTTTGATCTGAAACTG)3'

The abovementioned 18-mer(octadecamer) was prepared by the solid phase phosphoramidite method using an Applied Biosystems 381A DNA synthesizer under conditions recommended by the manufacturer. The product was analysed by ion-exchange high pressure liquid chromatography (HPLC) as described below; no further purification of the product was performed. The yield was 20 OD units (260nm) when dissolved in 1ml.

## Methods used for Examples 2 and 3

### DNA cleavage

In general the cleavage of about 1μg of plasmid DNA or DNA fragments was effected using about 5 units of a restriction enzyme (or enzymes) in about 20μl of an appropriate buffer solution.

### Generation of blunt ends:

If blunt ends were required they were produced by treating the DNA preparation with DNA Polymerase I, Klenow fragment (Molecular Cloning, A Laboratory Manual, Cold Spring Harbor Laboratory 1982).

5' end labelling of oligonucleotides:

Addition of radiolabelled phosphate groups to oligonucleotides was carried out as described (Maxam and Gilbert, Methods in Enzymology Vol.65 p499-560 Ed. Grossman and Moldave publisher Academic Press 1980).

Ligation of DNA Fragments:

Ligation reactions were carried out as described (Maniatis et al Molecular Cloning - A Laboratory Manual, Cold Spring Harbor Laboratory 1982).

Transformation of M13 DNA into bacterial cells was carried out using treatment with calcium chloride (Cohen et al 1973, P.N.A.S. 69, 2110).

Transformation of plasmid DNA into E. coli HB101 cells was as described by Hanahan (DNA Cloning Vol I Chapter 6, D.M. Glover ed. IRL Press, 1985) in Protocol 3 except that incubation with RFI was for 5 minutes.

Isolation of t-PA cDNA clones

RNA was isolated from the TRBM6 cell line (Browne et al 1985, Thrombosis and Haemostasis; 54; 422-424) using hot phenol/SDS extraction (M.R.D. Scott, 1982, Ph.D. Thesis, University of London) and messenger RNA purified using oligo dT cellulose chromatography. A TRBM6 cDNA library was established by synthesizing double-stranded cDNA using this messenger RNA essentially as described by M.R.D. Scott (Ph.D. Thesis 1982, University of London). This cDNA preparation was digested with BglII, size fractionated on a Biogel A150 column and ligated into a pAT153 vector which had been modified by introduction of a BglII linker at the NruI site; the resultant DNA clone was propagated in E. coli K12 DH1 cells. A t-PA-specific oligonucleotide probe (Browne et al, 1985; Gene 33, 279-284)was used to identify a t-PA cDNA clone in the cDNA library, this clone is known as pTR108, and carries a 2kb BglII fragment encoding the mature t-PA protein (Pennica et al, 1983, Nature, 301, 214-221).

A further cDNA clone λTR10, was isolated from a second TRBM6 cDNA library. The cDNA library was constructed using the λgt10 vector as described in 'DNA Cloning' Volume I, Chapters 2 and 3 (Ed. D.M. Glover (IRL Press, 1985)). The library was screened using appropriate t-PA specific oligonucleotide and plasmid probes using methods described previously (Browne et al, 1985, Gene, 33, 279-284). Clone λTR10 isolated from this library carries additional DNA 5' to that in pTR108, corresponding to the prepro-coding region and part of the 5' untranslated region (Pennica et al, 1983, Nature, 301, 214-221).

Growth of M13 single strand DNA:

A single M13 phage plaque was picked into a 1:100 dilution in 2YT (1.6% Bactotryptone, 1% Yeast extract, 1% NaCl) of a fresh overnight culture of E. coli strain BMH 71-18 (Gronenborn et al, 1976, Mol. Gen. Genet. 148, 243-250). The culture was grown at 37°C with shaking for 5-6 hours. The bacteria were pelleted and the supernatant retained. To this was added 200µl of 2.5M NaCl, 20% PEG6000 and the mixture was incubated at room temperature for 15 minutes. The mixture was centrifuged for 5 minutes in an Eppendorf microfuge and the resulting phage pellet was resuspended in 100µl of 10mM Tris pH 7.9, 0.1mM EDTA. After phenol extraction the phage DNA was precipitated with ethanol. The DNA pellet was washed with 70% ethanol, air dried and resuspended in 30µl of 10mM Tris pH 7.9, 0.1mM EDTA.

Growth of double stranded M13 DNA:

A single M13 phage plaque was picked into 1ml of 2YT and grown with shaking at 37°C for 6 hours. The bacteria were pelleted and the supernatant containing M13 phage retained. Meanwhile a one litre 1:100 dilution of an overnight culture of E. coli strain BMH 71-18 was grown at 37°C with shaking for 2 hours. 500µl of the M13 phage supernatant was added and the culture was shaken at 37°C for a further 4 hours. Preparation of double stranded DNA was carried out as described by Maniatis et al (Molecular Cloning, A Laboratory Manual, Cold Spring Harbor Laboratory, 1982).

Site directed mutagenesis:

The site-directed mutagenesis reactions were carried out essentially as described by Carter *et al* - (Oligonucleotide site-directed mutagenesis in M13, An Experimental Manual, Anglian Biotechnology Ltd., 1985).

The following priming mixture was set up in a total volume of 10µl: 1µg template DNA (mTR30 single stranded form, see Example 2); 3ng kinased mutagenic oligonucleotide primer (as described above); 1µl 10 × TM Buffer (100mM Tris pH 8.0, 100mM $MgCl_2$), 5.5µl $H_2O$. The mixture was sealed into a glass capillary, boiled for three minutes and allowed to cool to room temperature. To the priming mixture were then added:- 2µl 10 × TM buffer; 1µl of a mixture of 4mM each of dATP, dGTP, dCTP, dTTP; 1µl 5mM rATP; 1µl 100mM DTT; 12µl $H_2O$; 1 unit DNA Polymerase I (Klenow fragment); 7 units T4 DNA ligase giving a total final volume of 30µl. The mixture was incubated for 4h at 14°C.

The DNA was transformed in 3µl aliquots into *E. coli* strain BMH 71-18 mut L (Kramer *et al*, 1984, Cell 38, 879-887). The transformed bacteria were plated onto a lawn formed from *E. coli* strain BMH 71-18. Some of the plaques resulting from the transformations were picked and plated to form duplicate gridded arrays of bacterial colonies. These were lifted onto nitrocellulose and lysed as described (Grunstein and Hogness, 1975, P.N.A.S.72, 3961).

Screening conditions:

The nitrocellulose filters were prehybridised for 3h at 30°C in 6 × SSC (1 × SSC is 150mM NaCl, 15mM trisodium citrate) 0.1% SDS, 10 × Denhardt's (Denhardt's is 0.02% Polyvinyl-pyrrolidone, 0.02% Ficoll, 0.02% BSA), 50µg sonicated, heat denatured salmon sperm DNA. The filters were then mixed with radioactively labelled mutagenic primer under the same buffer conditions and were hybridised overnight at 30°C.

The filters were washed at a series of different temperatures in 6 × SSC plus 0.1% SDS: 15 minutes at 30°C; 3 minutes at 45°C; 3 minutes at 50°C. After each wash the filters were exposed, wet (with an intensifying screen) to Fuji RX-100 X-ray film.

Sequencing:

DNA sequencing was carried out by the dideoxy termination method (Sanger, Nicklen and Coulson, 1977, P.N.A.S. 74, 5463).

Plasmid preparation:

Large scale preparation of plasmid DNA and plasmid mini-preparations were carried out as described in Maniatis *et al* (Molecular Cloning - A Laboratory Manual, Cold Spring Harbor Laboratory, 1982).

Isolation of DNA fragments from low-melting-point (LMP) agarose gels

DNA fragments were isolated from LMP agarose gels as described by Maniatis *et al* (Molecular Cloning, A Laboratory Manual, 1982, Cold Spring Harbor).

Ligation of Synthetic Linkers to DNA

Synthetic linkers encoding restriction enzyme sites, were kinased and ligated to blunt-ended DNA as described by Maniatis *et al* (Molecular Cloning, A Laboratory Manual, 1982, Cold Spring Harbor).

Dephosphorylation of DNA

Vector DNA was dephosphorylated, where appropriate, by treatment with calf intestinal alkaline phosphatase as described by Maniatis et al (Molecular Cloning, A Laboratory Manual, 1982, Cold Spring Harbor).

Fibrin/agar overlay for detection of t-PA expression

Cell preparation: cells were trypsinised and plated out at $5.4 \times 10^5$ cells per 60mm dish and left 72h in growth medium (10% Serum, 1% stock solution of penicillin/streptomycin, 1% Glutamine in RPM1 1640 medium; Gibco, Paisley, Scotland) at 37°C in a humidified incubator in an atmosphere of 5% $CO_2$/95% air. The cells were then refed and were used for transfection after a further 24h.

Transfection procedure: The transfection, in Eagles MEM, used calcium coprecipitation as described in 'DNA Cloning' Ed. D.M. Glover (Chap. 15, C. Gorman). Glycerol shock and 5mM butyrate treatment were used.

Overlay: Agarose (Indubiose $A_{37}$), 2.4g in 95ml Eagles MEM (Gibco) heated to melting point in a bunsen flame, was then placed in a water bath maintained at 48°C. 5.6 ml of fibrinogen (20mg/ml) were diluted 1:1 with 5.6 ml of Eagles MEM (containing an extra 7 mg/ml of NaCl) and retained on ice. 3.3 ml of Eagles MEM (no additions) where aliquoted into a bijou containing 86µl of bovine thrombin at 50 NIH Units/ml (retained on ice). The cells were washed 3 times with Eagles MEM to remove serum. The thrombin and fibrinogen were warmed to 37°C in a water bath. 9.5 ml agarose were aliquoted into a pre-warmed universal. The thrombin was added to the agar, followed by the fibrinogen. The gel was poured over the cell layer and incubated at 37°C until lysis zones appeared.

Example 2A

Change in t-PA DNA sequence (1012-1014)

DNA coding for a novel, modified t-PA protein has been produced using the above mentioned oligonucleotide (Example 1) with sequence:-

5′ d(T C C T T T G A T C T G A A A C T G)3′

This oligonucleotide is complementary to nucleotides 1006-1023 inclusive of t-PA cDNA (Pennica et al, Nature, 1983, 301, 214-221) except for two mismatches at positions 1013 and 1014. The codon at position 1012-1014 has thus been changed from CGC to CAG. The resulting DNA sequence has been expressed in a eukaryotic cell system to give a fibrinolytically active protein.

All manipulation of the DNA referred to below was carried out as described in the Methods Section.

A cDNA clone coding for the protein t-PA was isolated from mRNA prepared from the TRBM6 cell line described in Thrombosis and Haemostasis, 1985, 54, 422-424. The 2kb BglII fragment encoding the mature t-PA cDNA sequence was isolated, blunt-ended and subcloned into M13 mp10 (Amersham International PLC Prod. No. N.4536) at the SalI site which had also been blunt-ended. Clones were isolated with the insert in both orientations. The two types of clone will henceforth be known as mTR10 and mTR20. One of these, mTR20, placed the t-PA cDNA insert 'in frame' and in the correct orientation, to allow expression of a t-PA fusion protein from the lacZ promoter in the M13 mp10 genome (Slocombe et al, 1982, P.N.A.S. 79, 5455-5459). This construct did not give good yields of either single or double stranded DNA.

The t-PA cDNA was put out of reading-frame by cutting double stranded DNA frcm the clone mTR10 with the enzymes BamHI and HindIII. The whole DNA mixture comprising the cleaved vector DNA and the t-PA insert DNA was blunt-ended and religated. The resulting construct was shown by DNA sequencing to have the following structure at the point of insertion indicating that the t-PA cDNA is out of reading-frame with respect to the M13 lacZ promoter.

mp10 derived sequence t-PA sequence

5' GGG GAT CAG CTT GGG CTG CAG GTC GA ↓GA TCT 3'

——————>

transcription BglII

This clone will be known as mTR30. Site directed mutagenesis was carried out on the clone mTR30 as described in Methods. After screening, five duplicate positive signals were selected. DNA sequencing was carried out on single stranded DNA from the five isolates and all clearly showed a changed DNA sequences. The sequence is 5'd(CAGTTTCAGATCAAAGGA)3' and the clone will be known as mTR60.

Example 2B

Transient Expression of modified t-PA DNA ($arg_{275} \rightarrow gln_{275}$) in Eukaryotic Cells

i) Vector Construction

The vector pRSV-β-globin (Gorman et al, Science, 221, 551-553, 1983) was modified to allow the insertion and expression of t-PA cDNA (see Fig.1). The β-globin sequences were removed by digesting 10µg of pRSV-β-globin DNA with 20 units each of HindIII and BglII for 2h and isolating the large DNA fragment from a 1% low melting point agarose gel as described in the methods section. The β-globin sequences were replaced with a DNA fragment encoding the 5' region of the cDNA from λTR10 which is a λgt10 cDNA clone ('DNA Cloning, Vol.I, chapters 2 and 3, Ed. D.M. Glover, IRL Press, 1985) constructed using TRBM6 cell messenger RNA (Thrombosis and Haemostasis, 1985, 54, 422-424). The cDNA insert in the λTR10 encodes the 5' untranslated region, prepro sequences and mature protein sequences up to the EcoRI site equivalent to base 801 (Pennica et al, Nature, 1983, 301, 214-221). The cDNA insert was cleaved from the vector by digestion of 5µg of DNA with 24 units of EcoRI for 1.5h. The DNA was then extracted with phenol/ chloroform, ethanol precipitated, resuspended, blunt-ended and ligated to kinased HindIII linkers as described in the Methods section. Ligation of linkers was at ambient temperature for 7 hours then overnight at 4°C. The DNA was then digested with 20 units of HindIII for 2 hours and a 800bp fragment corresponding to the HindIII-linkered t-PA cDNA was isolated after electrophoresis in a 1% low melting point agarose gel. This 800bp fragment was mixed with the large DNA fragment derived from pRSV-β-globin described above and ethanol precipitated. The DNA was resuspended and digested with 10 units each of HindIII and BglII for 1.7h, phenol/chloroform extracted, ethanol precipitated, resuspended and ligated as described in the methods section for 5h at ambient temperature and overnight at 4°C. 2µl of DNA was used to transform E. coli HB101 and colonies carrying plasmids containing the 5' end of the t-PA cDNA up to the BglII site equivalent to that numbered 187 in Pennica et al (Nature, 1983, 301, 214-221), were identified by HindIII/BglII digestion of plasmid mini-preps and the plasmid named pTRE1.

The sequences encoding the Rous sarcoma virus long terminal repeat (RSV-LTR), 5' region of t-PA cDNA, and Simian virus 40 (SV40) 3' sequences were then reconstructed in pAT153 in such a way that they formed a single XhoI fragment. Firstly, the RSV LTR was removed from pRSV-β-globin by digestion of 6µg DNA with 4U of SfaNI for 2.5h. The DNA was then phenol/chloroform extracted, ethanol precipitated, resuspended and blunt-ended. XhoI linkers were kinased and ligated to the DNA at ambient temperature for 8h and overnight at 4°C, then incubated at 70°C for 15 minutes to inactivate the ligase and ethanol precipitated. The linkered DNA was then resuspended and digested with 15 units each of HindIII and XhoI for 6h and a ~500 bp fragment corresponding to the RSV-LTR isolated after electrophoresis in a 1% LMP agarose gel. This DNA fragment was cloned into pAT153 prepared as follows: 6µg of pAT153 was digested with 30U of EcoRI for 2h, then phenol/chloroform extracted and ethanol precipitated. The DNA was resuspended, blunt-ended, ligated to XhoI linkers, resuspended and digested with HindIII and XhoI as above. A ~3.4kb fragment was isolated after electrophoresis in a 1% LMP agarose gel and ethanol precipitated with the ~500bp fragment above. The DNA was ligated to give pTREO.

The ~0.2kb fragment encoding the 5' part of the t-PA cDNA was isolated from pTRE1 by digesting 10µg of DNA with 30 units each of HindIII and BglII for 2 hours and the fragment isolated after electrophoresis in a 1% LMP agarose gel. This fragment was cloned into pAT153 prepared as follows: 5µg

of pAT153 was digested to completion with BamHI. Following phenol/chloroform extraction and ethanol precipitation, the DNA was resuspended and blunt-ended then ligated to kinased BglII linkers. The DNA was phenol/chloroform extracted, ethanol precipitated, resuspended and digested with 30 units each of HindIII and BglII for 3h. The large DNA band was isolated after electrophoresis in a 1% LMP agarose gel, ethanol precipitated with the ~0.2kb fragment above and ligated together to give pTRE8.

A DNA fragment encoding the SV40 sequences from pTRE1 was isolated as follows: 5µg of pTRE1 was digested with 60 units of EcoRI for 2h, blunt-ended and ligated to XhoI linkers as described previously. After phenol/chloroform extraction and ethanol precipitation, the DNA was resuspended and digested sequentially with BglII and XhoI. A ~1.6kb band encoding the SV40 sequences was isolated after electrophoresis in a 1% LMP agarose gel and cloned into pTRE8 prepared as follows: 5µg of pTRE8 was digested with 15 units of SalI for 3½h, blunt-ended and ligated to XhoI linkers as described previously. Following phenol/chloroform extraction and ethanol precipitation, the DNA was resuspended and digested sequentially with 30 units of BglII for 1h and 10 units XhoI for 2h. The large DNA band was isolated after electrophoresis in a 1% LMP agarose gel, ethanol precipitated with the~1.6kb fragment above and ligated to give pTRE11.

A ~2kb DNA fragment encoding the 5′ t-PA cDNA sequences, SV40 sequences and part of pAT153 were cleaved from pTRE11 by digestion of 5µg DNA with 30 units HindIII and 6 units XmaIII for 6h. After electrophoresis in a 1% LMP agarose gel a ~ 2kb band was isolated and cloned into pTREO prepared as follows: 4.8µg pTREO was digested with HindIII and XmaIII as above and the large DNA fragment isolated after electrophoresis in a 1% LMP agarose gel. This fragment was ethanol precipitated with the ~ 2kb fragment above and ligated to give pTRE12.

t-PA cDNA BglII fragments were cloned into the unique BglII site of pTRE12 as shown in Figure 2. pTRE7 contains a ~ 2kb BglII fragment, encoding the mature t-PA protein and part of the 3′ untranslated region, derived from a cDNA library produced from TRBM6 cells, Thrombosis and Haemostasis, 1985, 54, 422-424), cloned into the unique BglII site of pTRE1. 5µg of pTRE7 was digested to completion with BglII and the ~2kb fragment isolated after electrophoresis in a 1% LMP agarose gel. This was ethanol precipitated and ligated with pTRE12, previously digested with BglII and phosphatased as described in the methods section, to give pTRE15 which expresses wild-type t-PA. mTR60 was digested with BglII and a ~2kb fragment isolated and cloned into pTRE12 as described above to give pTRE32 which expresses mutated t-PA.

ii) Fibrin/agar overlay

Expression of modified and unmodified t-PA from transfected human Hela cells (24 hours post-transfection) was detected using the fibrin/agarose overlay technique as described in the Methods section. Lytic zones were obtained from pTRE15 (unmodified t-PA) and pTRE32 (modified t-PA) transfected cells, but not from cells transfected with the empty parent vector pTRE12 (t-PA ).

## Example 3

Another mutated form of t-PA has previously been described in which the growth factor domain has been deleted; amino acids 51-87 inclusive (EP-A-0207589). An altered form of t-PA is described here which combines both the mutation described in EP-A-0207589 and that of present Example 2B. The expression plasmid encoding this modified t-PA was constructed as follows.The approximately 900bp NarI-SstI fragment carrying the arg275 → gln275 mutation was excised from pTRE32 described above and the approximately 410bp HindIII-NarI fragment excised from plasmid pTRE24 described in EP-A-0207589. These two fragments were ligated with the approx. 6kb HindIII-SstI fragment of pTRE15 (EP-A-0207589) to form plasmid pTRE48.

Plasmid DNA prepared from recombinant E. coli by standard techniques was used to transfect cultured human Hela cells by the calcium phosphate precipitation technique essentially as described in 'High Efficiency Gene Transfer into Mammalian Cells' by C. Gorman (In 'DNA Cloning Volume II', ed. D. Glover, IRL Press Ltd. 1985).

Expression of modified and unmodified t-PA from transfected human Hela cells (24 hours post-transfection) was detected using the fibrin/agarose overlay technique as described in Methods section. Lytic zones were obtained, indicating the presence of a plasminogen activator.

In the figures:

Figure 1 (a, b and c): Construction of vector (pTRE12) for insertion and expression of t-PA sequences

Abbreviations: λTR10 - λgt10 clone containing the 5′ part of t-PA cDNA

LTR - Rous sarcoma virus long terminal repeat

β -Rabbit β globin cDNA

SV - Simian virus 40 sequences including small t antigen intron and early region polyadenylation site

5′ -5′ part of t-PA cDNA (approximately 0.2kb long) with 3′ end equivalent to the BglII site at position 187 in Pennica et al Nature 301, 214-221, 1983

B - recognition site for BamHI

Bg - recognition site for BglII

E - recognition site for EcoRI

H - recognition site for HindIII

S - recognition site for SalI

Sf - recognition site for SfaNI

X - recognition site for XhoI

Xm - recognition site for XmaIII

Figure 2: Construction of vectors expressing wild-type or mutant t-PA (pTRE15 and pTRE32 respectively)

Abbreviations are as in Figure 1. t-PA wild type is cDNA encoding normal t-PA, t-PA mutant is cDNA encoding altered t-PA ($arg_{275} \rightarrow gln_{275}$).

**Claims**

1. A fibrinolytically active tissue-type plasminogen activator which has been modified:
(a) in the region of the growth factor domain, and
(b) to provide an amino acid other than arginine or lysine at position 275.

2. A modified tissue-type plasminogen activator according to claim 1, in which the modification (a) is in the region from amino acid residues 51 to 87 inclusive.

3. A modified tissue-type plasminogen activator according to of claim 1 or 2, wherein the modification (a) comprises the deletion of all or part of the growth factor domain.

4. A modified tissue-type plasminogen activator according to claim 3, wherein the modification (a) comprises the deletion of amino acid residues 51 to 87 inclusive.

5. A modified tissue-type plasminogen activator according to any preceding claim, wherein the modification (b) provides an amino acid selected from histidine, threonine, alanine, glycine, serine, asparagine, aspartic acid, glutamine and glutamic acid at position 275.

6. A modified tissue-type plasminogen activator according to any preceding claim, in which the amino acid at position 275 is glutamine.

7. A modified tissue-type plasminogen activator prepared according to Example 3.

8. A derivative of the modified tissue-type plasminogen activator according to any of claims 1 to 7.

9. A DNA polymer comprising a nucleotide sequence that encodes a modified tissue-type plasminogen activator according to any of claims 1 to 7.

10. A replicable expression vector capable, in a host cell, of expressing a DNA polymer according to claim 9.

11. A host cell transformed with the vector according to claim 10.

12. A pharmaceutical composition comprising modified tissue-type plasminogen activator according to any of claims 1 to 8 in combination with a pharmaceutically acceptable carrier.

13. A modified tissue-type plasminogen activator according to any of claims 1 to 8 for use as an active therapeutic substance.

14. A modified tissue-type plasminogen activator according to any of claims 1 to 8 for use in the treatment of thrombotic disease.

15. Use of a modified tissue-type plasminogen activator according to any of claims 1 to 8 for the preparation of a medicament for the treatment of thrombotic disease.

16. A process for preparing modified tissue-type plasminogen activator according to claim 1, which process comprises expressing DNA encoding said modified tissue-type plasminogen activator in a recombinant host cell and recovering the modified tissue-type plasminogen activator product.

17. A process for preparing a DNA polymer according to claim 9, by the condensation of appropriate mono-, di-or oligomeric nucleotide units.

18. Modified tissue-type plasminogen activator obtainable by the process of claim 16.

Claims for the following Contracting State: ES.

1. A process for preparing a fibrinolytically active tissue-type plasminogen activator which has been modified:

(a) in the region of the growth factor domain, and

(b) to provide an amino acid other than arginine or lysine at position 275, and in which any catalytic site essential for fibrinolytic activity is optionally blocked by a removable blocking group, which process comprises expressing DNA encoding said modified tissue-type plasminogen activator in a recombinant host cell and recovering the modified tissue-type plasminogen activator product, and thereafter as necessary, optionally blocking the catalytic site with a removable blocking group.

2. A process according to claim 1, in which the modification (a) is in the region from amino acid residues 51 to 87 inclusive.

3. A process according to claim 1 or 2, wherein the modification (a) comprises the deletion of all or part of the growth factor domain.

4. A process according to claim 3, wherein the modification (a) comprises the deletion of amino acid residues 51 to 87 inclusive.

5. A process according to any preceding claim, wherein the modification (b) provides an amino acid selected from histidine, threonine, alanine, glycine, serine, asparagine, aspartic acid, glutamine and glutamic acid at position 275.

6. A process according to any preceding claim, in which the amino acid at position 275 is glutamine.

7. A process for preparing a DNA polymer comprising a nucleotide sequence that encodes a modified tissue-type plasminogen activator as defined in any of claims 1 to 6, by the condensation of appropriate mono-, di-or oligomeric nucleotide units.

8. A process for preparing a replicable expression vector capable, in a host cell, of expressing a DNA polymer as defined in claim 7, which process comprises cleaving a vector compatible with said host cell to provide a linear DNA segment having an intact replicon, and combining the said linear segment with one or more DNA molecules which, together with said linear segment, encode the modified tissue-type plasminogen activator, under ligating conditions.

9. A process for preparing a host cell transformed with a replicable expression vector as defined in claim 8, which process comprises transforming a host cell with said vector under transforming conditions.

10. Use of a modified tissue-type plasminogen activator as defined in any of claims 1 to 6 for the preparation of a medicament for the treatment of thrombotic disease.

11. A method of treating thrombotic diseases, which comprises administering to the sufferer an effective non-toxic amount of modified tissue-type plasminogen activator according to claim 1.

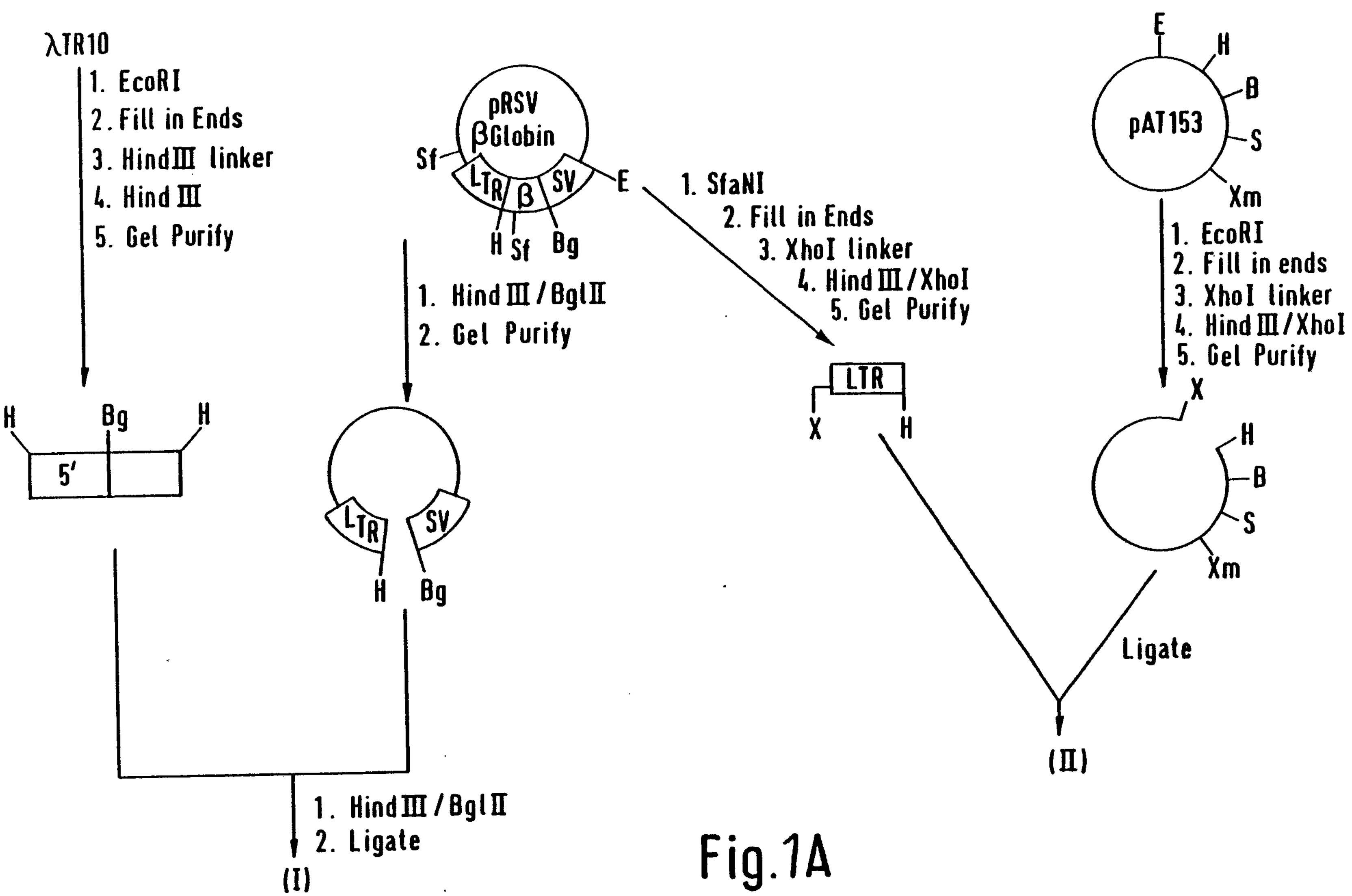
λTR10
1. EcoRI
2. Fill in Ends
3. HindIII linker
4. Hind III
5. Gel Purify
H
Bg
H
5′
pRSV
βGlobin
Sf
LTR
β
SV
E
H
Sf
Bg
1. Hind III / BglII
2. Gel Purify
LTR
SV
H
Bg
1. HindIII / BglII
2. Ligate
(I)
1. SfaNI
2. Fill in Ends
3. XhoI linker
4. Hind III / XhoI
5. Gel Purify
LTR
X
H
pAT153
E
H
B
S
Xm
1. EcoRI
2. Fill in ends
3. XhoI linker
4. Hind III / XhoI
5. Gel Purify
X
H
B
S
Xm
Ligate
(II)

Fig.1A

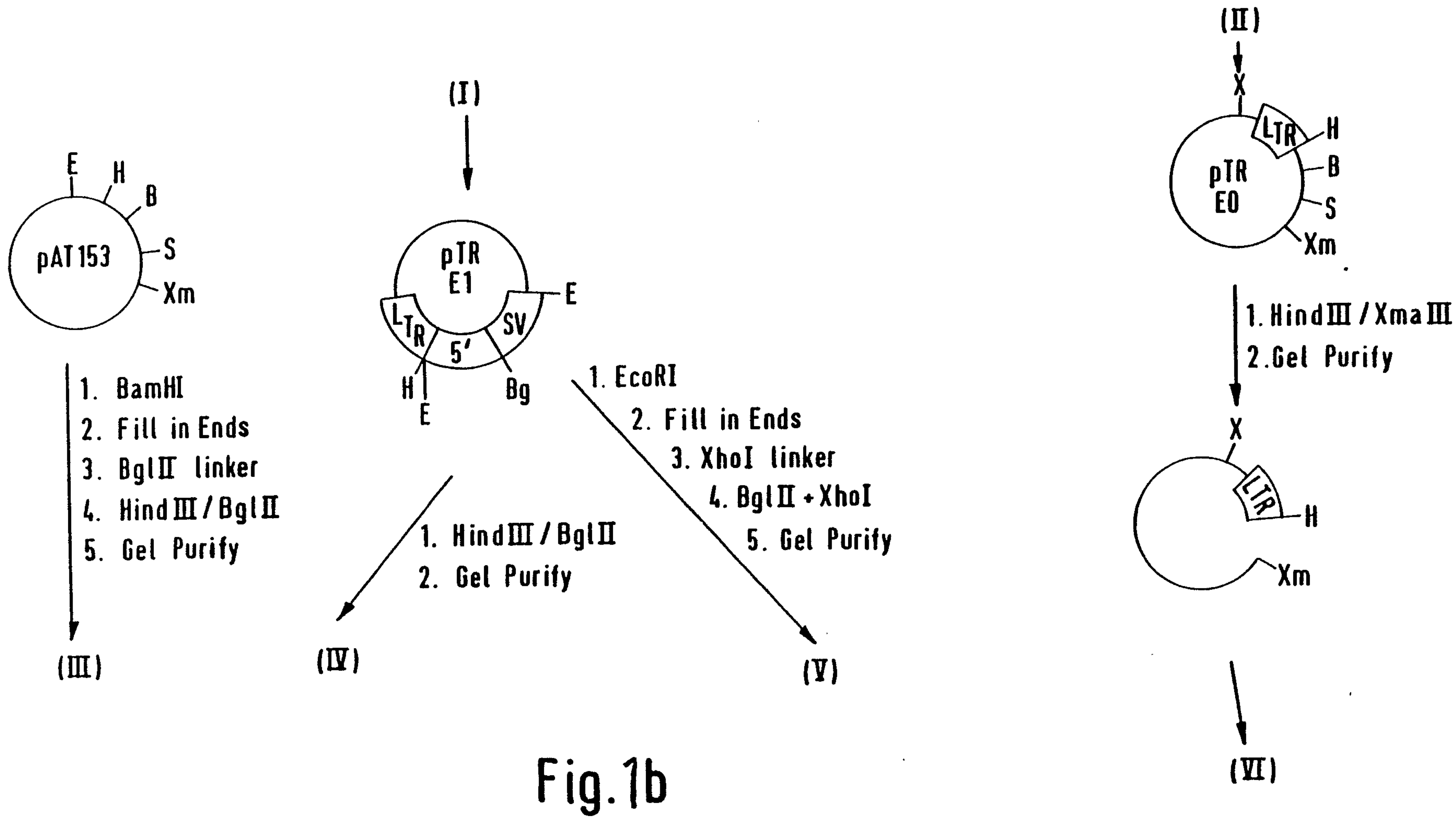
pAT153
E
H
B
S
Xm
1. BamHI
2. Fill in Ends
3. BglII linker
4. HindIII / BglII
5. Gel Purify
(III)
(I)
pTR
E1
LTR
5'
SV
H
E
Bg
E
1. HindIII / BglII
2. Gel Purify
(IV)
1. EcoRI
2. Fill in Ends
3. XhoI linker
4. BglII + XhoI
5. Gel Purify
(V)
(II)
X
LTR
H
B
S
Xm
pTR
E0
1. HindIII / XmaIII
2. Gel Purify
X
LTR
H
Xm
(VI)

Fig.1b

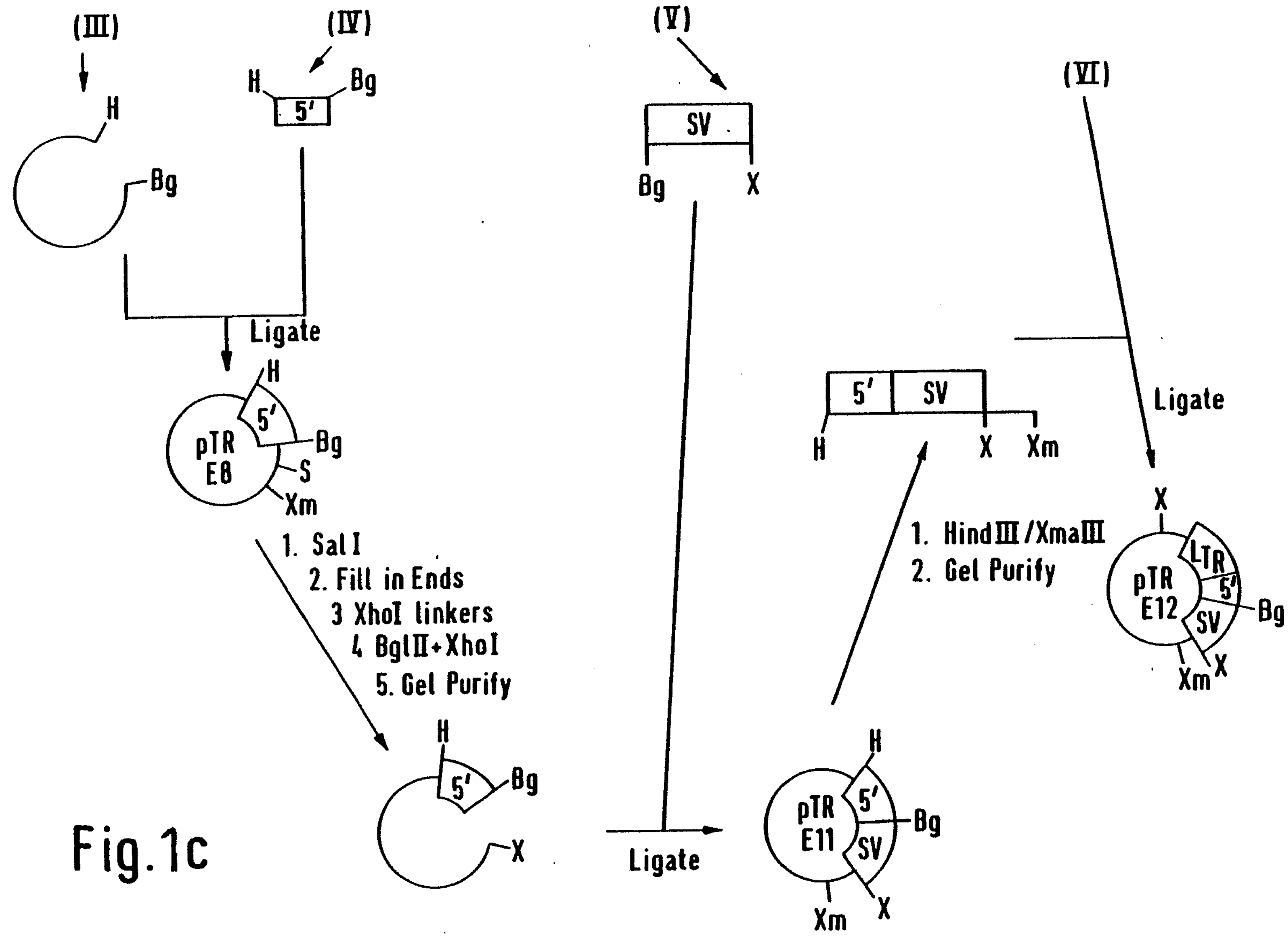
(III)
H
Bg
(IV)
H
5'
Bg
(V)
SV
Bg
X
(VI)
Ligate
pTR E8
H
5'
Bg
S
Xm
1. Sal I
2. Fill in Ends
3 XhoI linkers
4 BglII+XhoI
5. Gel Purify
H
5'
Bg
X
Ligate
pTR E11
H
5'
Bg
SV
X
Xm
1. HindIII/XmaIII
2. Gel Purify
5'
SV
H
X
Xm
Ligate
pTR E12
X
LTR
5'
Bg
SV
X
Xm

Fig.1c

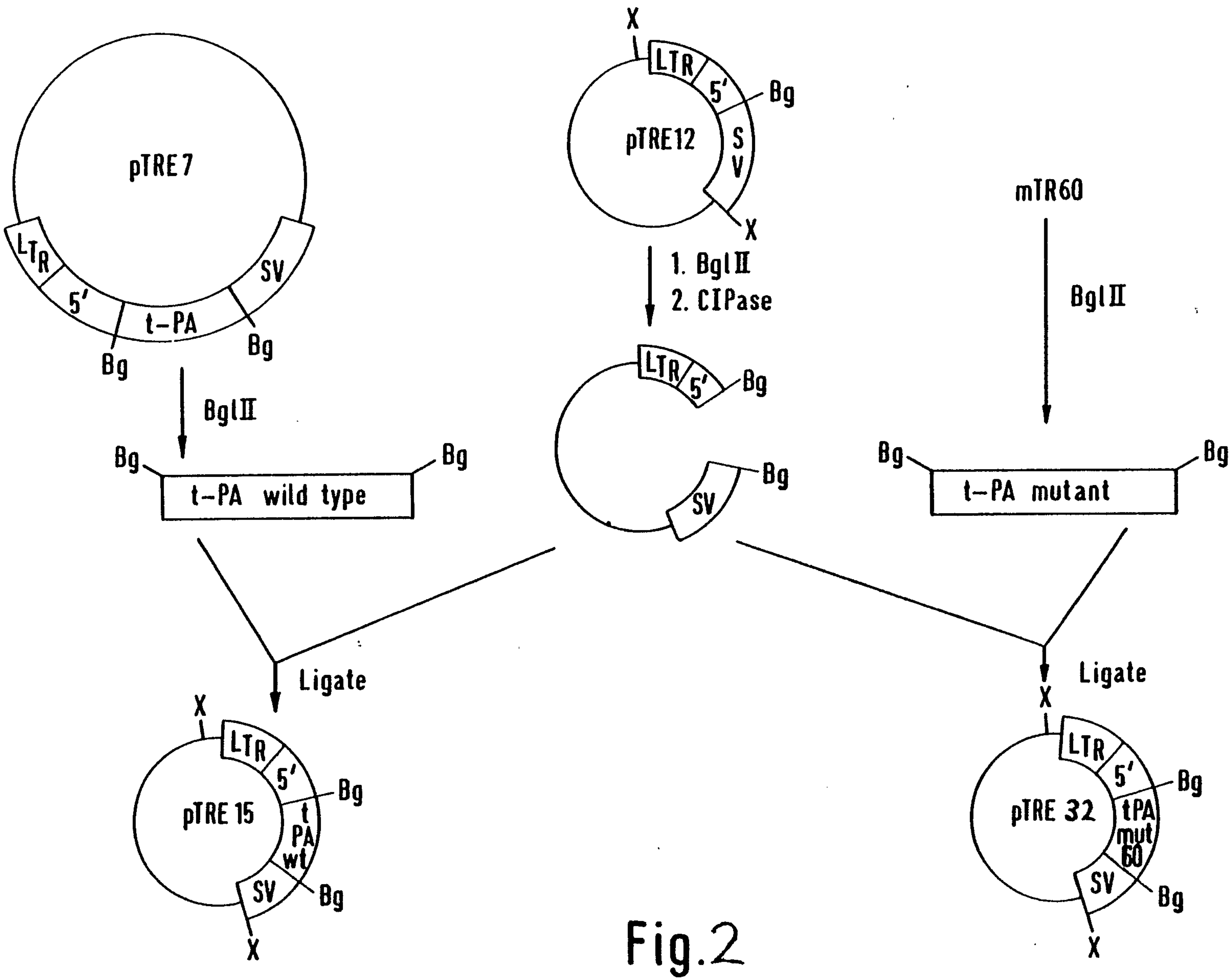
pTRE7
LTR
5'
t-PA
SV
Bg
Bg
BglII
Bg
t-PA wild type
Bg
pTRE12
X
LTR
5'
Bg
S
V
X
1. BglII
2. CIPase
LTR
5'
Bg
SV
Bg
mTR60
BglII
Bg
t-PA mutant
Bg
Ligate
X
LTR
5'
Bg
pTRE 15
t
PA
wt
SV
Bg
X
Ligate
X
LTR
5'
Bg
pTRE 32
tPA
mut
60
SV
Bg
X

Fig.2